# EUROPEAN PATENT APPLICATION

(11) **EP 1 574 581 A2**
(43) Date of publication of application: **14.09.2005**
(21) Application number: 05075548.7
(22) Date of filing: 07.03.2005
(51) Int. Cl.: C12P 5/02

(54) **Method and apparatus for preparing methane gas**

(30) Priority: 08.03.2004 NL 1025653
(71) Applicant: E.M. ENGINEERING F.T.S. B.V., NL-5995 SB Kessel (NL)
(72) Inventor: van Staveren, Nicolaas Arie, 5995 SB Kessel (NL)
(74) Representative: Valkonet, Rutger

(57) **Abstract**

The invention relates to a method for preparing methane gas from a CO₂-containing starting material. It is an object of the present invention to provide a method and apparatus for preparing methane gas, wherein a CO₂ containing starting material is converted into a high-energy gas flow that contains methane gas in an environmentally friendly manner.

## Description

The present invention relates to a method and apparatus for preparing methane gas from a CO₂ containing starting material.

Carbon dioxide gas is released in large quantities upon combustion of fossil fuels such as oil and coal. Many scientists hold carbon dioxide responsible for the process of global heating, which will eventually cause the ice caps on the poles to melt and the sea level to rise. Recent reports suggest that a rise of the sea level of 50 centimetres to a few metres can be expected in the century to come. Such a rise of the sea level means that low-lying countries, such as the Netherlands, will have to take drastic measures, such as raising the dikes in coastal areas and setting up large-scale water control projects inland. In addition to that, factory farming has resulted in the production of a waste gas which, in addition to methane and hydrogen sulphide, also contains carbon dioxide gas. The methane that is present in such biogas is a high-energy component, which can be used for generating durable energy by combusting the methane gas. In many cases, however, the methane content is too low to use the biogas as a fuel.

It is an object of the present invention to provide a method and apparatus for preparing methane gas, wherein a CO₂ containing starting material is converted into a high-energy gas flow that contains methane gas in an environmentally friendly manner.

Another object of the present invention is to provide a method and apparatus for preparing methane gas, which apparatus is suitable for processing CO₂ containing starting materials having a low CO₂ content.

Yet another object of the present invention is to provide a method and apparatus for preparing methane gas, wherein a methane gas containing starting material that is directly suitable for energy generation is obtained.

The present invention as referred to in the introduction is characterized in that the CO₂ containing starting material is supplied to a reactor together with hydrogen gas, in which reactor microorganisms are present, which microorganisms produce methane gas from CO₂ and hydrogen.

Making use of microorganisms, a conversion of CO₂ and hydrogen takes place, with methane gas being formed according to a natural process, thereby accomplishing one or more of the aforesaid objects.

It is in particular desirable to operate the reactor under a light (absolute) overpressure of 1,5-3 bar, which process conditions cause the reaction equilibrium in the reactor to be advantageously shifted to the intended formation of methane gas.

The microorganisms that are present in the reactor, in particular the microorganisms of the methanobacterium-thermoautotrophicum type, are preferably immobilised on a solid substrate material, so that a stable conversion of CO₂ and hydrogen into methane gas is ensured.

It is in particular preferable to supply the CO₂ containing starting material and hydrogen gas at the bottom side of the reactor, in which case the methane gas that is formed is discharged at the upper side of the reactor. The advantage of such a manner of supplying is that an intense contact is effected between CO₂, hydrogen and the microorganisms, which has an advantageous effect on the conversion of CO₂ and hydrogen into methane. In addition, in such an embodiment the residence time of the CO₂ containing starting material and hydrogen gas can be adjusted to achieve the intended conversion values.

The CO₂ containing starting material and the hydrogen gas being supplied at the bottom side of the reactor are converted into methane in the reactor, and the gas flow that exits the reactor at the upper side will be composed of an amount of unconverted CO₂, unconverted hydrogen and methane that has been formed, plus any inevitable impurities, such as sulphur containing compounds. The hydrogen in said gas flow is recovered in that the gas flow that exits the reactor is supplied to a separation device, such as a membrane reactor, with the comparatively small hydrogen molecules passing the pores of the membrane and the comparatively large molecules of the other gas components being rejected by the membrane, The hydrogen-rich gas flow is subsequently led back to the bottom side of the reactor. The gas flow obtained from the separation device can thus be considered to be a methane-rich gas flow supplemented with unconverted CO₂ and inevitable impurities.

The temperature of the reactor is set to obtain optimum conditions for the formation of methane gas, in which connection in particular a temperature range of 40-80 °C, preferably 60-70 °C, is used.

In a special embodiment, the hydrogen gas that is supplied to the reactor has been obtained through electrolysis of water, which process produces oxygen in addition to hydrogen. The obtained hydrogen, supplemented with a particular amount of unconverted hydrogen from the aforesaid separation device, is supplied to the reactor.

Preferably, the CO₂ containing starting material that is supplied to the reactor has a CO₂ content of at least 80%. more preferably at least 95%, the remaining amount consisting of inevitable impurities and CH₄, in which connection it should be noted, however, that the CO₂ containing starting material must not contain any components that have an adverse effect on the biological conversion that takes place in the reactor, for example by poisoning the microorganisms that are present in the reactor. For example, if a CO₂ containing starting material having a CO₂ content of, for example, 30-70 % is available, it is desirable to increase the CO₂ content thereof to the desired percentage as mentioned above. Especially biogas is suitable for use as a special CO₂ containing starting material, which biogas is subjected to a pre-purification step so as to obtain a CO₂ containing starting material whose CO₂ content is higher than before the purification of the biogas, The methane gas obtained in such a pre-purification step is advantageously reused, for example by combining it with the gas flow that exits the separation device.

In a special embodiment it is desirable to carry out said pre-purification step by contacting the biogas with a sorption medium that selectively sorbs CO₂ from the biogas that is supplied, which sorption medium is subsequently subjected to temperature and pressure conditions such that a starting material rich in CO₂ is obtained, which is subsequently supplied to the reactor.

According to a special embodiment of the present invention, said pre-purification step is carried out by contacting the biogas with a sorption medium that selectively sorbs components other than CO₂ from the biogas that is being supplied, after which the thus pre-purified biogas is supplied to the reactor as a starting material that is rich in CO₂, Although so far biogas has been mentioned as a suitable CO₂ containing starting material, it should be understood that also exhaust gases from a combustion engine can be used as a CO₂ containing starting material and be subjected to a pre-purification step, if desired.

The present invention further relates to an apparatus for preparing methane gas from a CO₂ containing starting material, which apparatus is provided with the necessary supply and discharge lines, pumps and compressors, said apparatus comprising a reactor to which a supply line for the CO₂ containing starting material and a supply line for the hydrogen gas are connected, as well as a discharge line for the methane gas that is formed. Special embodiments of the present apparatus are defined in the appended claims.

The present invention will now be explained in more detail by means of an example and with reference to a figure, in which connection it should be noted, however, that the present invention is by no means limited to such a specific description.

A CO₂ containing starting material, for example biogas, is supplied to a compressor 2 at numeral 1, after which a pre-purification step is carried out in the sorption reactors 3, 4. Present in said sorption reactors 3, 4 is a sorption medium, which selectively binds CO₂, for example. By passing the CO₂ containing starting materials through the sorption reactors 3. 4, the sorption medium will selectively remove CO₂ from the gas flow being supplied, so that no CO₂ will be detected at the discharge lines 7, 9. The obtained gas flow is discharged via numeral 23. When the sorption medium is saturated with CO₂, the supply of CO₂ containing starting material is stopped and such conditions are created in the reactors 3, 4, for example by releasing pressure, that a starting material rich in CO₂ is obtained, which is subsequently supplied to the reactor 13 via the compressor 11. By alternately supplying the reactors 3, 4 with a CO₂ containing starting material it becomes possible to continuously supply a starting material rich in CO₂ to the reactor 13. By periodically operating the reactors 3, 4 in this manner a possibility has been provided to supply the reactor 13 with a continuous gas flow, with the valves 5, 6, 7, 8, 9 and 10 being opened and/or closed in a logical sequence. It is also possible, however, to provide the sorption reactors 3, 4 with a sorption medium that selectively binds gases other than CO₂, so that a starting material rich in CO₂ is constantly supplied via the compressor 11. The reactor 13, in which microorganisms are present that convert CO₂ and hydrogen into methane, is maintained at the temperature required for conversion by using a water jacket. Hydrogen gas is supplied to the bottom side of the reactor 13 via numeral 12, and a gas flow is withdrawn from the reactor 13 via numeral 15, which gas flow is supplied to the separation device 17, in particular a membrane installation. The hydrogen-rich gas flow thus obtained is supplied to the reactor 13 anew via numeral 12. The methane-rich gas flow, which further contains inevitable impurities and CO₂, is discharged via numeral 24, possibly combined with gas flow 23, and advantageously reused, for example as a combustion gas for generating electricity. Hydrogen gas is obtained by feeding the electrolysis device 19 with an aqueous flow 20, and the oxygen that is formed is discharged. If desired, recirculation of biomass may take place in the reactor 13, in which case the biomass that is withdrawn at the bottom side of the reactor 13 is fed back to the reactor 13 at the upper side thereof via numeral 14.

Using the present invention, it has thus appeared to be possible to convert a CO₂ containing starting material into a methane gas containing product that can be advantageously reused. In addition, the harmful emission of carbon dioxide into the atmosphere is reduced, because the CO₂ containing starting material is converted into the intended methane gas in a reactor as a result of the presence of certain microorganisms,

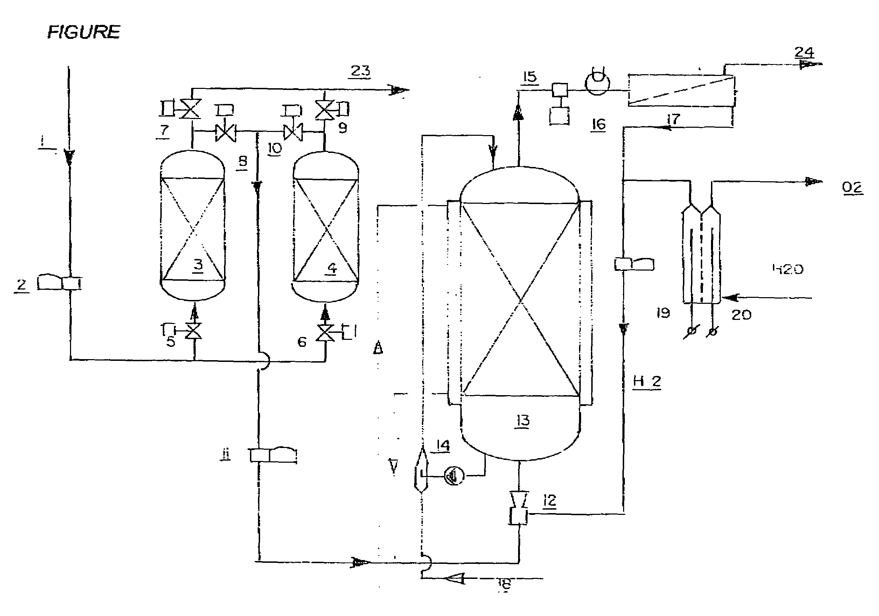

## Claims

1. A method for preparing methane gas from a CO₂-containing starting material, **characterized in that** the CO₂ containing starting material is supplied to a reactor together with hydrogen gas, in which reactor microorganisms are present, which microorganisms produce methane gas from CO₂ and hydrogen.

2. A method according to claim 1, **characterized in that** the reactor is operated under a light (absolute) overpressure of 1.5-3 bar.

3. A method according to any one or more of the preceding claims, **characterized in that** the microorganisms are immobilised on a solid substrate material,

4. A method according to any one or more of the preceding claims, **characterized in that** the CO₂ containing starting material and hydrogen gas are supplied at the bottom side of the reactor, with the methane gas that is formed being discharged at the upper side of the reactor.

5. , A method according to claim 4, **characterized in that** the gas flow that exits the reactor is supplied to a separation device, after which separation device a hydrogen-rich gas flow is led back to the bottom side of the reactor.

6. A method according to any one or more of the preceding claims, **characterized in that** the temperature of the reactor ranges between 40-80 °C.

7. A method according to claim 6, **characterized in that** the temperature of the reactor ranges between 60-70 °C.

8. A method according to any one or more of the preceding claims, **characterized in that** said microorganisms are of the methanobacterium-thermoautotrophicum type.

9. A method according to any one or more of the preceding claims, **characterized in that** hydrogen gas is obtained through electrolysis of water.

10. A method according to any one or more of the preceding claims, **characterized in that** biogas is used as the CO₂ containing starting material.

11. A method according to claim 10, **characterized in that** said biogas is subjected to a pre-purificatian step so as to obtain a CO₂ containing starting material whose CO₂ content is higher than before the purification of the biogas.

12. A method according to claim 11, **characterized in that** said pre-purification step is carried out by contacting the biogas with a sorption medium that selectively sorbs CO₂ from the biogas that is supplied, which sorption medium is subsequently subjected to temperature and pressure conditions such that a starting material rich in CO₂ is obtained, which is subsequently supplied to the reactor.

13. A method according to claim 11, **characterized in that** said pre-purification step is carried out by contacting the biogas with a sorption medium that selectively sorbs components other than CO₂ from the biogas that is supplied, after which the thus pre-purified biogas is supplied to the reactor as a starting material that is rich in CO₂,

14. A method according to any one of the claims 1-9, **characterized in that** exhaust gases from a combustion engine are used as a CO₂ containing starting material.

15. A method according to any one or more of the preceding claims, **characterized in that** nutrients for the microorganisms are supplied to the reactor.

16. Apparatus for preparing methane gas from a CO₂ containing starting material, which apparatus is provided with the necessary supply and discharge lines, pumps and compressors, **characterized in that** the apparatus comprises a reactor to which a supply line for the CO₂ containing starting material and a supply line for the hydrogen gas are connected, as well as a discharge line for the methane gas that is formed.

17. Apparatus according to claim 16, **characterized in that** the discharge line for the methane gas that is formed is connected to a separation device, after which the hydrogen that is present in the methane gas is connected to the supply line for hydrogen gas to the reactor via a discharge line.

18. Apparatus according to claim 16, **characterized in that** a pre-purification plant is interposed in the supply line for the CO₂ containing starting material. which pre-purification plant comprises a number of sorption reactors, in which a gas flow supplied via a supply line is pre-purified so as to increase the CO₂ content thereof.
